# EUROPEAN PATENT SPECIFICATION

(11) **EP 2 964 189 B1**
(45) Date of publication and mention of the grant of the patent: **26.06.2019**
(21) Application number: 13776659.8
(22) Date of filing: 30.09.2013
(51) Int. Cl.: A61K 9/00, A61K 9/06, A61K 31/60

(54) **SALICYLIC ACID GEL**
SALIZYLSÄUREGEL
GEL D'ACIDE SALICYLIQUE

(30) Priority: 08.03.2013 US 201313789780
(43) Date of publication of application: 13.01.2016
(73) Proprietor: Vantage Specialty Ingredients, Inc., Warren, NJ 07059 (US)
(72) Inventor: GERLACH, Chris, Buford, Georgia 30519 (US); DAVIES, Mike, Dacula, Georgia 30019 (US)
(74) Representative: Sobisch, Peter
(86) International application number: PCT/US2013/062561
(87) International publication number: WO 2014/137395

(56) References cited:
- EP-A1- 0 052 705
- EP-A1- 1 795 181
- WO-A1-98/52927
- WO-A2-00/02593

## Description

### CROSS-REFERENCE OF RELATED APPLICATIONS

This application claims priority to U.S. Application Serial No. 13/789,780 filed on March 8, 2013, hereby incorporated by reference in its entirety.

### FIELD OF THE INVENTION

The invention relates to a gel of salicylic acid, and products produced therefrom.

### BACKGROUND OF THE INVENTION

Salicylic acid, also known as 2-hydroxybenzenecarboxylic acid, is a monohydroxybenzoic acid. Its salts and esters are known as salicylates. Salicylic acid has the formula:

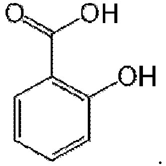

Salicylic acid is also known for providing pain relief when applied as a liniment, for example

Salicylic acid is used in many skin-care products. For example, salicylic acid is well known for its use in anti-acne treatments. In addition to the treatment of acne, salicylic acid is also used in products for treatment of psoriasis, calluses, corns, keratosis pilaris, and warts. It works as a keratolytic, bacteriocide and comedolytic agent. Salicylic acid is also used in shampoos for treatment of dandruff and as a chemical exfoliant.

Salicylic acid can cause burns if applied in high concentrations. Typically, over-the-counter limits are 2% for topical treatments (that remain on the skin) and 3% for cleansers or shampoo (products that are washed off.) Higher concentrations (e.g. up to 40 wt %) may be used for wart removal but should be applied cautiously and only to the wart and not the surrounding skin.

Salicylic acid is poorly soluble in water. It is therefore difficult to prepare solutions of salicylic acid that remain precipitate-free.

### BRIEF SUMMARY OF THE INVENTION

The present invention is directed to a salicylic acid gel consisting of salicylic acid and a stabilizer compound, wherein the salicylic acid is present in at least 1 wt% based on total weight of the gel. The present invention is further directed to a salicylic acid solution comprising the salicylic acid gel and a solvent. The present invention is further directed to products prepared with the salicylic acid gel or with dilute solutions of the gel.

The present invention is further directed to a salicylic acid gel comprising salicylic acid and a stabilizer compound, wherein the salicylic acid comprises at least 30 wt%, based on total weight of the gel. The present invention is further directed to a salicylic acid solution comprising the salicylic acid gel and a solvent. The present invention is further directed to products prepared with the salicylic acid gel or with dilute solutions of the gel in solvent. The present invention is further directed to a salicylic acid gel consisting of salicylic acid and a stabilizer compound, wherein the salicylic acid comprises at least 30 wt%, based on total weight of the gel.

In further aspects, the salicylic acid gel prepared in accordance with any aspect above is combined with ingredients to form various products including body or hand lotions, anti-dandruff shampoos, wart medications, and anti-acne medications.

Further aspects are directed to making a solution or skin care product comprising mixing the salicylic acid gel in accordance with any aspect defined above with a solvent.

### DETAILED DESCRIPTION OF THE INVENTION

Aspects of the invention are directed to a gel of salicylic acid and at least one stabilizer compound selected from nitrogen compounds. One aspect is particularly directed to a concentrated gel. The concentrated gel allows relatively large amounts of salicylic acid to be shipped and stored in a safe and stable manner. A higher concentration of salicylic acid further allows smaller shipping and storage space. Another aspect is directed to gel consisting of salicylic acid and at least one stabilizer compound selected from nitrogen compounds. This gel also allows salicylic acid to be shipped and stored in a safe and stable manner. In addition, the gel is flowable so is easier to manufacture and use.

In one aspect, a (concentrated) salicylic acid gel comprises at least 30 wt% salicylic acid, at least 35 wt%, or at least 40 wt% salicylic acid gel, for example 30 wt% to 60 wt% salicylic acid, or 35 wt% to 55 wt%, or 40 to 55 wt% salicylic acid each based on total weight of the gel. Specific amounts may be 30 wt%, 35 wt%, 40 wt%, 50 wt%, and 60 wt% based on total weight of the gel based on total weight of the gel.

The concentrated salicylic acid gel comprises the stabilizer compound in an amount of at least 40 wt%, at least 45 wt%, at least 60 wt%, and up to 70 wt%, for example 40 to 70 wt%, or 45 to 60 wt%, each based on total weight of the gel which is an amount effective to provide stability to the salicylic acid in the gel. Specific amounts may be 40 wt%, 50 wt%, 60 wt%, 65 wt%, and 70 wt% based on total weight of the gel.

In one aspect, the concentrated salicylic acid gel is formed by mixing salicylic acid with the stabilizer compound until the salicylic acid is dissolved in the stabilizer compound. In a particular aspect, no water or solvent such as ethanol is included in the gel. In a further particular aspect, no other active ingredient is present in the gel. In a particular aspect, there are only two components that form the concentrated gel: the stabilizer compound (complexing agent) and the salicylic acid. That is the salicylic acid gel consists of salicylic acid and a stabilizer compound, wherein the salicylic acid comprises at least 30 wt%, based on total weight of the gel.

The mixing of the salicylic acid and the stabilizer compound may be done at room temperature. Alternatively the mixing may occur at an elevated temperature such as up to 50° C. Generally mixing takes about 30 to 120 minutes.

The concentrated gel may then be stored for future use. A benefit of the concentrated gel is that such gel contains very concentrated amounts of salicylic acid, and less storage space is required than dilute solutions of salicylic acid. The gel is storage stable for at least 2 years.

The stabilizer compound may be any suitable nitrogen compound that stabilizes the salicylic acid in the concentrated gel. Nitrogen compounds useful to form the highly concentrated salicylic gel include, but not limited to, alkoxylated amides, alkoxylated amines, alkyl-substituted amino acids, alkylamido alkylamines, amides, amine oxides, amines, and betaines. Ideally, a clear product should be produced.

In one aspect, the nitrogen compound is cocamidopropyl dimethylamine. The cocamidopropyl dimethylamine is particularly suitable for high concentrations of salicylic acid. When diluted in water, the resulting solution is stable and clear.

The concentrated gel can be diluted to any suitable level for use. Dilution of the salicylic acid gel may occur by the addition of water, a short chain alcohol such as ethanol and isopropanol, or any combination thereof. Upon dilution, the salicylic acid forms a clear, stable solution in the water or alcohols - that is, the salicylic acid does not precipitate out.

In particular the dilute solutions comprise wherein the concentration of the salicylic acid in the solution is at least 0.5 wt%, at least 1 wt%, at least 5 wt%, at least 10 wt%, at least 15 wt% or at least 20 wt% each based on total weight of the solution.

The present invention is further directed to products prepared with the salicylic acid gel or with dilute solutions of the gel in solvent wherein the concentration of the salicylic acid in the product is at least at least 0.5 wt%, at least 1 wt%, at least 5 wt%, at least 10 wt%, at least 15 wt%, at least 20 wt%, at least 30 wt%, or at least 40 wt% each based on total weight of the product.

Other aspects of the invention are directed to a gel consisting of salicylic acid and at least one stabilizer compound selected from nitrogen compounds. The gel allows salicylic acid to be shipped and stored in a safe and stable manner.

The salicylic acid gel consists of at least 1 wt%, at least 5 wt%, at least 10 wt%, at least 15 wt%, or at least 20 wt%, salicylic acid based on total weight of the gel. Specific amounts may be, but not limited to, 1wt%, 5 wt% 10 wt%, or 20 wt%.

The salicylic acid gel consists of the stabilizer compound in an amount of at least 30 wt%, at least 80 wt%, at least 90 wt%, at least 95 wt%, at least 99 wt%, and up to 99 wt%, for example between 70 wt% and 99 wt%.

In one aspect, the salicylic acid gel is formed by mixing salicylic acid with the stabilizer compound until the salicylic acid is dissolved in the stabilizer compound. No water or solvent such as ethanol is included in the gel. The mixing of the salicylic acid and the stabilizer compound may be done at room temperature. Alternatively the mixing may occur at an elevated temperature such as up to 50° C. Generally mixing takes about 3 to 120 minutes. The gel may be stored.

The stabilizer compound may be any suitable nitrogen compound that stabilizes the salicylic acid in the gel as discussed above and incorporated by reference herein.

As discussed above, the gel can be diluted to any suitable level for use such as by the addition of water, a short chain alcohol such as ethanol and isopropanol, or any combination thereof. In particular the dilute solutions comprise wherein the concentration of the salicylic acid in the solution is at least 0.5 wt%, at least 1 wt%, at least 5 wt%, at least 10 wt%, at least 15 wt% or at least 20 wt% each based on total weight of the solution. Upon dilution, the salicylic acid forms a clear, stable solution in the water or alcohols.

The present invention is further directed to products prepared with the salicylic acid gel or with dilute solutions of the gel in solvent wherein the concentration of the salicylic acid in the product is at least at least 0.5 wt%, at least 1 wt%, at least 5 wt%, at least 10 wt%, at least 15 wt% or at least 20 wt% each based on total weight of the product.

For pH evaluation, 95 wt% water was added to 5 wt% of the concentrated gel (40 wt% salicylic acid, 60 wt% cocamidopropyl dimethylamine) resulting in a 2 wt% salicylic acid concentration; the maximum allowed for anti-acne products. The same evaluation was run using 92.5 wt% water and 7.5 wt% of the gel (resulting in 3 wt% active salicylic acid; the maximum allowed for anti-dandruff products) and 40 wt% water and 60 wt% of the gel (resulting in 24 wt% active salicylic acid) to evaluate for the wart remover monograph. All of these studies resulted in clear, stable solutions with pH levels between 3 and 4.

The pH of the dilute system is generally less than 5, such as 2 to 5, 2.5 to 4.5, typically 3 to 4.

For topical purposes, the dilute system must pass the USP Monograph for a Salicylic Gel which includes an assay for salicylic acid.

The diluted product may be combined with other suitable ingredients to form the final products such as creams, lotions, make-ups, toners, astringents, skin cleansing compositions, shampoos, and conditioners. These compositions contain about 0.1-40 wt% of salicylic acid. The amount of salicylic acid in the final product depends on the intended purpose of the product.

Creams typically contain about 10-90 wt% water and 10-90 wt% oil. Creams may also contain humectants, emollients, surfactants, emulsifiers, preservatives and fragrances. Creams would generally contain from 0.1 to 5 wt% salicylic acid.

Lotions typically contain 20-80 wt% oil and 10-80 wt% water in an emulsion form. In addition, lotions may contain humectants, emollients, surfactants, fragrances, preservatives and so forth. Creams would generally contain from 0.2 to 5 wt% salicylic acid.

Make-ups typically contain about 5-70 wt% oil, 10-95 wt% water, and about 5-40 wt% pigment. In addition, the makeup may contain surfactants, silicones as part of the oil phase, humectants, emollients, preservatives, fragrances, etc. Make-up would generally contain from 0.1 to 3 wt% salicylic acid.

Anti-dandruff shampoos typically contain 1-40 wt% of a cleansing surfactant and 10-90 wt% water. The shampoo may also contain any one of ingredients such as surfactants, colorants, preservatives, fragrance, emulsifiers, viscosity adjusters, and conditioning agents. Anti-dandruff shampoos would generally contain from 0.18 to 3 wt% salicylic acid.

Hair conditioners typically contain include 10-95 wt% water, 0.5-30 wt% conditioning ingredients such as quaternary ammonium compounds or amphoteric polymers, proteins, etc., and 1-40% surfactants. Hair conditioners may also contain volatile or nonvolatile silicones. Hair conditioners would generally contain from 0.1 to 4 wt% salicylic acid.

Toners typically contain about 0-85 wt% alcohol, 0.01-5 wt% surfactant, and 0.1-5 wt% humectants, 0.1-85% water.

The salicylic acid may also be used in ointments, gels, or solutions. Suitable ointments are hydrophilic ointments (USP) or petroleum.

The amount of salicylic acid present in the final product depends on the product. For example, acne treatment products generally contain 0.5 to 2 wt% salicylic acid, dandruff and seborrheic dermatitis, and psoriasis treatment products generally contain 3 wt% salicylic acid, and wart treatments generally contain up to 40 wt% salicylic acid, typically 5 wt% to 40 wt% or 17 wt% to 25 wt%.

### Example 1

A 40% salicylic acid was blended with 60% cocamidopropyl dimethylamine. The resulting concentrated gel was slightly viscous, clear, and yellow.

The concentrated gel was diluted to 2 wt% active salicylic acid in water and separately in ethanol. The resulting solutions were clear and colorless. No precipitate was formed either in water or in ethanol. The pH of the water solution was approximately 3.2.

Stability tests were then performed on the water solution. The concentrated gel and the diluted 2 wt % active salicylic acid solution were prepared and subjected to accelerated stability protocol which consisted of five freeze/thaw cycles and two weeks in a 50°C oven. Under both of these conditions there were no significant changes to either sample. The samples that were frozen were obviously solid when removed from the freezer and the 50°C samples were less viscous. In both cases, when the samples returned to room temperature, they were essentially identical to the control samples. There was no color or viscosity change and no precipitate was formed.

The gel was also diluted to 25 wt% active salicylic acid in water. The resulting solution was a slightly viscous, yellow, clear solution. No precipitate was formed. A physical accelerated stability test was run on this prototype consisting of samples be held at 50°C for two weeks and another sample run through five freeze/thaw cycles. Under both of these conditions there were no physical changes to the product include pH, viscosity, color and appearance.

### Example 2

A lotion was prepared with 5% of the gel of example 1 in 91% water with 4% Egel 305 [Polyacrylamide (&) C12-13 isoparaffin (&) Laureth-7]. The result was a white lotion that would be applicable for an anti-acne product. It underwent the same accelerated stability testing as mentioned above (50°C for 2 weeks and 5 freeze/thaw cycles); there were no significant physical changes to the product during the stability testing.

### Example 3

An anti-dandruff shampoo was prepared containing 3 wt% salicylic acid.

| | | Percent |
|---|---|---|
| | Water | 36.50 |
| Active ingredient | Cocamidopropyl Dimethylamine (&) | 7.50 |
| | Salicylic Acid | |
| | (Curcylic® 40) | |
| Surfactant blend | PEG-80 Sorbitan Laurate (&) | 50.00 |
| | Cocamidopropyl Betaine (&) Sodium | |
| | Trideceth Sulfate (&) Glycerin (&) Disodium | |
| | Lauroamphodiacetate (&) PEG-150 | |
| | Distearate (&) Sodium Laureth-13 | |
| | Carboxylate | |
| | (Sulfochem™ B-NBB) | |
| Thickener | PEG-120 Methyl Glucose Trioleate (&) | |
| | Propylene Glycol (&) Water | |
| | (Glucamate™ LT) | 5.00 |
| Preservative | Propylene Glycol (&) Diazolidinyl Urea (&) | 1.00 |
| | Methylparaben (&) Propylparaben | |
| | (Nipaguard PDU) | |
| | Citric Acid | qs to pH 4 |
| | | 100.00% |

In an appropriate container, water, and Curcylic®40 were mixed until uniform. Sulfochem B-NBB, Glucamate LT and Nipaguard PDU were added and mixed until homogenous. Then citric acid was added, with continued mixing, until the batch reached pH of approximately 4.0. Viscosity: >1300cp. Stability: Passed 2 weeks 50°C; 5 Freeze/ Thaw cycles.

### Example 4

A wart remover was prepared containing _% salicylic acid.

| | | Percent |
|---|---|---|
| | Water | 46.5 |
| Active Ingredient | Cocamidopropyl Dimethylamine (&) | 42.5 |
| | Salicylic Acid | |
| | (Curcylic® 40) | |
| | Propanediol | 5.0 |
| | (Zemea®) | |
| | Hydrogenated Methyl Abeitate | 1.0 |
| | (Meristant® 101L) | |
| | Alcohol | 4.0 |
| Surfactant Blend | Phenoxyethanol (&) Methylparaben (&) | 1.0 |
| | Ethylparaben (&) Butylparaben (&) | |
| | Propylparaben (&) Isobutylparaben | |
| | (Phenonip) | |
| | Citric Acid | Qs |
| | | 100.0% |

In an appropriate container, water and Curcylic® 40 were blended until homogenous. In a separate container, alcohol and Meristant® 101L were mixed until homogenous and then added to and mixed with the Curcylic®/Water blend. Zemea® and Phenonip were then added to the blend and mixed until clear. The pH was adjusted with citric acid to 4-4.5 if necessary.

## Claims

1. A composition in the form of a stable gel and containing, based on the total weight of the gel:
(A)a salicylic acid which is poorly soluble in water in an amount of at least 30 wt. %, at least 35 wt. % , or 40 wt. % and
(B)a liquid nitrogen-containing compound which functions as a stabilizer (hereafter for convenience "stabilizer") in an amount of at least 30 wt. %, at least 90 wt. %, at least 95 wt. % and up to 99 wt. %, wherein said stabilizer and the amount thereof function to form said stable gel by solubilizing and dissolving said salicylic acid.

2. The composition according to claim 1 wherein the salicylic acid is present in an amount of 30 wt. % to 60 wt. % or 35 wt. % to 55 wt. % or 40 wt. % to 55 wt. %.

3. The composition according to claim 1 or 2, wherein the stabilizer is present in an amount of at least 70 wt. % to 99 wt. %.

4. The composition according to any one of claims 1 to 3, wherein the stabilizer is selected from the group consisting of alkoxylated amides, alkoxylated amines, alkyl-substituted amino acides, alkylamido alkylamines, amides, amine oxides amines and betaines.

5. The composition according to any one of claims 1 to 4 wherein the stabilizer is cocamidopropyl dimethylamine (hereinafter cocamine).

6. A composition according to any one of claims 1 to 5, wherein no solvent is present in the gel or including also a solvent.

7. A composition according to any one of claims 1 to 6 and including also a solvent in which the gel is dissolved , the solvent being water, ethanol, or isopropyl alcohol or other short chain alcohol.

8. A composition according to claim 7 and **characterized in that** the solvent is water and the pH of the solution is less than 5 or is 2 to 5, or is 2,5 to 4,5 or is 3 to 4.

9. A composition which consists essentially of a clear, stable, dilute solution of salcylic acid and which is **characterized by** being prepared by adding a solvent to a stable gel of any one of claims 1 to 4 or 6 to 9.

10. A composition according to claim 6 including a solvent and **characterized in that** the concentration of the salicylic acid in the dilute solution is at least 0,5 wt. %, at least 1 wt. %, at least 5 wt. %, at least 10 wt. % or at least 15 wt. % or at least 20 wt. % or no greater than 20 wt. %, each based on the total weight of the solution.

11. A skin-care product comprising the salicylic acid gel or solution as defined in any preceding claim **characterized in that** the amount of salicylic acid in the product is in the range of 0.1 wt. % to 40 wt. % or is at least 0,5 wt. %, at least 1 wt. %, at least 5 wt. %, at least 10 wt. %, at least 15 wt. % or at least 20 wt. % each based on the total weight of the product.

12. A skin-care product according to claim 10 or 11 **characterized in that** the product is a cream, body or hand lotion, make-up, toner, astringent, skin-cleaning composition, shampoo, anti-dandruff shampoo, hair conditioner, or a product for the treatment of warts, acne, seborrheic dermatitis or psoriasis.

13. An acne-treatment product according to claim 12 in which the concentration of of the salicylic acid is 0,5 wt. % to 2 wt. % based on the total weight of the acne treatment product.

14. A wart treatment product according to claim 11 in which the concentration of the salicylic acid is 5 wt. % to 40 wt. % or 17 wt. % to 25 wt. % based on the total weight of the wart treatment product.

15. A skin-care product according to claim 11 in the form of a lotion and in which the concentration of the salicylic acid is 0,2 wt. % to 5 wt. % based on the total weight of the product.

16. A skin-care product according to claim 11 in the form of an anti-dandruff shampoo and in which the concentration of the salicylic acid is 0,18 wt. % to 3 wt. % based on the total weight of the product.

17. A method of making a salicylic acid solution comprising combining the salicylic acid gel according to any one of claims 1 to 6 with a solvent and mixing.

18. A method of making a skin-care product which comprises a plurality of ingredients comprising combining the ingredients with the salicylic acid solution of claim 17 and mixing.

19. A method which is effective for preparing a skin-care product that contains a plurality of ingredients and which includes combining the ingredients with the salicylic acid solution of claim 17 or with the salicylic acid gel of claim 6 which does not contain a solvent and in which the amount of salicylic acid and stabilizer each comprises at least 30 wt. % of the total weight of the solution or of the gel.

20. A method according to claim 19 wherein the amount of salicylic acid is 30 wt. % to 60 wt. % or 40 wt. % to 55 wt. % based on the total weight of the solution or of the gel.

21. A method according to claim 19 or 20 wherein the stabilizer is cocamine.

22. A method according to claim 21, wherein the amount of cocamine is 40 wt. % 70 wt. % or 45 wt. % to 60 wt. & based on the total weight of the solution or the gel.

23. A skin-care product made according to the method of claim 19.

24. A skin-care product according claim 23 selected from the group consisting of a cream, a body lotion, a hand lotion, an anti-dandruff shampoo, a wart medication, an anti-acne medication, a make-up, a toner, an astringent, a skin cleansing composition, a shampoo, a conditioner, an ointment, a product to treat seborrheic dermatitis and a psoriasis treatment product and based on the weight of the product:
a) a cream containing 0,1 wt. % to 5 wt.% of salicylic acid;
b) a lotion containing 0,2 wt. % to 5 wt. % of salicylic acid;
c) a make-up containing 0,1 wt. % to 3,0 wt. % of salicylic acid;
d) an anti-dandruff shampoo containing 0,1 wt. % to 4 wt. % of salicylic acid;
e) a hair-conditioner containing 0,1 wt. % to 4 wt. % of salicylic acid
f) an acne-treatment medication containing 0,5 wt. % to 2 wt. % of salicylic acid;
g) a wart medication containing 5 wt. % to 40 wt. % of salicylic acid or 17 wt. % to 25 wt. % of salicylic acid.

## Patentansprüche

1. Eine Zusammensetzung in der Form eines stabilen Gels, welche bezogen auf das Gesamtgewicht des Gels aus
(A) einer in Wasser schlecht löslichen Salicylsäure in einer Menge von wenigstens 30 Gew.-%, wenigstens 35 Gew.-% oder 40 Gew.-% und
(B) einer flüssigen Stickstoff enthaltenden Verbindung besteht, welche als Stabilisator (hiernach üblicherweise "Stabilisator" genannt) wirkt, und zwar in einer Menge von wenigstens 30 Gew.-%, wenigstens 90 Gew.-% und bis zu 99 Gew.-%, wobei der Stabilisator und dessen Menge dahingehend wirken, dass das genannte stabile Gel gebildet wird, indem die genannte Salicylsäure löslich gemacht und aufgelöst wird.

2. Die Zusammensetzung nach Anspruch 1, wobei die Salicylsäure in einer Menge von 30 Gew.-% bis 60 Gew.-% oder von 35 Gew.-% bis 55 Gew.-% oder von 40 Gew.-% bis 55 Gew.-% vorhanden ist.

3. Die Zusammensetzung nach Anspruch 1 oder 2, wobei der Stabilisator in einer Menge von wenigstens 70 Gew.-% bis 99 Gew.-% vorhanden ist.

4. Die Zusammensetzung nach einem der Ansprüche 1 bis 3, wobei der Stabilisator aus einer Gruppe ausgewählt ist, die aus alkoxylierten Amiden, alkoxylierten Aminen, alkylsubstituierten Aminosäuren, Alkylamidoalkylaminen, Amiden, Aminoxiden, Aminen und Betainen besteht.

5. Die Zusammensetzung nach einem der Ansprüche 1 bis 4, wobei der Stabilisator aus Cocamidopropyldimethylamin besteht, (im folgenden Cocamin genannt)

6. Die Zusammensetzung nach einem der Ansprüche 1 bis 5, wobei in dem Gel kein Lösungsmittel vorhanden ist oder wobei auch ein Lösungsmittel vorhanden ist.

7. Die Zusammensetzung nach einem der Ansprüche 1 bis 6 welche auch ein Lösungsmittel enthält, in dem das Gel gelöst ist, wobei das Lösungsmittel aus Wasser, Ethanol, oder Isopropylalkohol oder einem anderen kurzkettigen Alkohol besteht.

8. Die Zusammensetzung nach Anspruch 7, **dadurch gekennzeichnet, dass** das Lösungsmittel aus Wasser besteht und der pH-Wert der Lösung weniger als 5 oder 2 bis 5 oder 2,5 bis 4,5 oder 3 bis 4 beträgt.

9. Die Zusammensetzung, welche im wesentlichen aus einer klaren, stabilen, verdünnten Lösung aus Salicylsäure besteht und welche **dadurch gekennzeichnet ist dass** sie durch Hinzufügung eines Lösungsmittels zu einem stabilen Gel nach einem der Ansprüche 1 bis 4 oder 6 bis 8 hergestellt worden ist.

10. Die Zusammensetzung nach Anspruch 6, welche ein Lösungsmittel umfasst und **dadurch gekennzeichnet ist, dass** die Konzentration der Salicylsäure in der verdünnten Lösung wenigstens 0,5 Gew.-%, wenigstens 1 Gew.-%, wenigstens 5 Gew.-%, wenigstens 10 Gew.-% oder wenigstens 15 Gew.-% oder wenigstens 20 Gew.-% oder nicht mehr als 20 Gew.% beträgt, jeweils bezogen auf das Gesamtgewicht der Lösung.

11. Ein Hautpflegeprodukt, welches aus dem Salicylsäuregel oder der Lösung nach einem der vorangegangenen Ansprüche besteht, **dadurch gekennzeichnet, dass** die Menge an Salicylsäure in dem Produkt in dem Bereich von 0,1 Gew.-% bis 40 Gew.-%, wenigstens 0,5 Gew.-%, wenigstens 1 Gew.-%, wenigstens 5 Gew.-%, wenigstens 10 Gew.-%, wenigstens 15 Gew.-% oder wenigstens 20 Gew.% beträgt, jeweils bezogen auf das Gesamtgewicht des Produktes.

12. Ein Hautpflegeprodukt nach Anspruch 10 oder 11, **dadurch gekennzeichnet, dass** das Produkt eine Creme, eine Body Lotion, eine Handcreme, ein Makeup, ein Toner, ein Adstringens, eine Zusammensetzung zur Hautreinigung, ein Shampoo, ein Anti-Schuppen-Shampoo, ein Mittel zur Haarspülung, oder ein Produkt zur Behandlung von Warzen, Aknen, seborrhoischer Dermatitis oder Psoriasis ist.

13. Ein auf die Behandlung von Aknen ausgerichtetes Produkt nach Anspruch 12, wobei die Konzentration an Salicylsäure 0,5 Gew.-% bis 2 Gew.-% bezogen auf das Gesamtgewicht des Behandlungsprodukts beträgt.

14. Ein auf die Behandlung von Warzen ausgerichtetes Produkt nach Anspruch 11, wobei die Konzentration der Salicylsäure 5 Gew.-% bis 40 Gew.-% oder 17 Gew.-% bis 25 Gew.-% bezogen auf das Gesamtgewicht des Behandlungsprodukts beträgt.

15. Ein Hautpflegeprodukt nach Anspruch 11 in der Form einer Lotion, wobei die Konzentration an Salicylsäure 0,2 Gew.-% bis 5 Gew.-% bezogen auf das Gesamtgewicht des Produktes beträgt.

16. Ein Hautpflegeprodukt nach Anspruch 11 in der Form eines Anti=Schuppen-Shampoos, wobei die Konzentration an Salicylsäure 0,18 Gew.-% bis 3 Gew.-% bezogen auf das Gesamtgewicht des Produktes beträgt.

17. Ein Verfahren zur Herstellung einer Salicylsäurelösung, wobei das Salicylsäuregel nach einem der vorangegangenen Ansprüche 1 bis 6 mit einem Lösungsmittel zusammengefasst und gemischt wird.

18. Ein Verfahren zur Herstellung eines Hautpflegeproduktes bestehend aus einer Vielzahl von Bestandteilen, wobei die Bestandteile mit der Salicylsäurelösung gemäß Anspruch 17 zusammengefasst und gemischt werden.

19. Ein Verfahren zur Zubereitung eines Hautpflegeproduktes, welches eine Vielzahl von Bestandteilen enthält, wobei die Bestandteile mit der Salicylsäurelösung gemäß Anspruch 17 oder mit dem Salicylsäuregel gemäß Anspruch 6, welches kein Lösungsmittel enthält, zusammengefasst wird, wobei die Menge an Salicylsäure und an Stabilisator jeweils wenigstens 30 Gew.-% des Gesamtgewichts der Lösung oder des Gels beträgt.

20. Verfahren nach Anspruch 19, wobei die Menge an Salicylsäure 30 Gew.-% bis 60 Gew.-% oder 40 Gew.-% bis 55 Gew.-% bezogen auf das Gesamtgewicht der Lösung oder des Gels beträgt.

21. Verfahren nach Anspruch 19 oder 20, wobei der Stabilisator aus Cocamin besteht.

22. Verfahren nach Anspruch 21, wobei die Menge an Cocamin 40 Gew.-%, 70 Gew.-% oder 45 Gew.-% bis 60 Gew.-% bezogen auf das Gesamtgewicht der Lösung oder des Gels beträgt.

23. Ein Hautpflegeprodukt, hergestellt nach dem Verfahren gemäß Anspruch 19.

24. Ein Hautpflegeprodukt gemäß Anspruch 23, ausgewählt aus der aus einer Creme, einer Body Lotion, einer Handcreme, einem Anti-Schuppen-Shampoo, einer Warzenmedikation, einer Anti-Aknen-Medikation, einem Makeup, einem Toner, einem Adstringens, einer Zusammensetzung zur Hautreinigung, einem Shampoo, einem Mittel zur Haarspülung, einer Salbe, einem Produkt zur Behandlung seborrhoischer Dermatitis und einem Mittel zur Behandlung von Psoriasis bestehenden Gruppe und welches bezogen auf das Gewicht des Produktes
a) bei einer Creme 0,1 Gew.-% bis 5 Gew.-% an Salicylsäure enthält;
b) bei einer Lotion 0,2 Gew.-% bis 5 Gew.-% an Salicylsäure enthält;
c) bei einem Makeup 0,1 Gew.-% bis 3 Gew.-% an Salicylsäure enthält;
d) bei einem Anti-Schuppen-Shampoo 0,1 Gew.-% bis 4 Gew.-% an Salicylsäure enthält;
e) bei einem Mittel zur Haarspülung 0,1 Gew.-% bis 4 Gew.-% an Salicylsäure enthält;
f) bei einem Mittel zur Behandlung von Aknen 0,5 Gew.-% bis 2 Gew.-% an Salicylsäure enthält;
g) bei einem Mittel zur Behandlung von Warzen 5 Gew.-% bis 40 Gew.-% oder 17 Gew.-% bis 25 Gew.-% an Salicylsäure enthält.

## Revendications

1. Composition sous la forme d'un gel stable et contenant, sur la base du poids total du gel :
(A) un acide salicylique qui est faiblement soluble dans l'eau en une quantité d'au moins 30 % en poids, d'au moins 35 % en poids, ou de 40 % en poids et
(B) un composé azoté liquide qui joue le rôle de stabilisateur (ci-après « stabilisateur » pour plus de commodité) en une quantité d'au moins 30 % en poids, d'au moins 90 % en poids, d'au moins 95 % en poids et jusqu'à 99 % en poids, dans laquelle ledit stabilisateur et la quantité de celui-ci servent à former ledit gel stable par la solubilisation et la dissolution dudit acide salicylique.

2. Composition selon la revendication 1 dans laquelle l'acide salicylique est présent en une quantité de 30 % en poids à 60 % en poids ou de 35 % en poids à 55 % en poids ou de 40 % en poids à 55 % en poids.

3. Composition selon la revendication 1 ou 2, dans laquelle le stabilisateur est présent en une quantité d'au moins 70 % en poids à 99 % en poids.

4. Composition selon l'une quelconque des revendications 1 à 3, dans laquelle le stabilisateur est sélectionné dans le groupe constitué par les amides alcoxylés, les amines alcoxylées, les acides aminés substitués avec un alkyle, les alkylamido alkylamines, les amides, les oxydes d'aminé, les amines et les bétaïnes.

5. Composition selon l'une quelconque des revendications 1 à 4 dans laquelle le stabilisateur est la cocamidopropyl diméthylamine (ci-après cocamine).

6. Composition selon l'une quelconque des revendications 1 à 5, dans laquelle aucun solvant n'est présent dans le gel ou incluant également un solvant.

7. Composition selon l'une quelconque des revendications 1 à 6 et incluant également un solvant dans lequel le gel est dissous, le solvant étant l'eau, l'éthanol, ou l'alcool isopropyle ou un autre alcool à chaîne courte.

8. Composition selon la revendication 7 et **caractérisée en ce que** le solvant est l'eau et le pH de la solution est inférieur à 5 ou est de 2 à 5, ou est de 2,5 à 4,5 ou est de 3 à 4.

9. Composition qui consiste essentiellement en une solution diluée, stable et limpide d'acide salicylique et qui est **caractérisée en ce qu'**elle est préparée par l'ajout d'un solvant à un gel stable selon l'une quelconque des revendications 1 à 4 ou 6 à 9.

10. Composition selon la revendication 6 incluant un solvant et **caractérisée en ce que** la concentration de l'acide salicylique dans la solution diluée est au moins de 0,5 % en poids, au moins de 1 % en poids, au moins de 5 % en poids, au moins de 10 % en poids ou au moins de 15 % en poids ou au moins de 20 % en poids ou au plus de 20 % en poids, chacun étant basé sur le poids total de la solution.

11. Produit de soin pour la peau comprenant le gel ou la solution d'acide salicylique tel que défini dans une quelconque revendication précédente **caractérisé en ce que** la quantité d'acide salicylique dans le produit est située dans la plage allant de 0,1 % en poids à 40 % en poids ou est au moins de 0,5 % en poids, au moins de 1 % en poids, au moins de 5 % en poids, au moins de 10 % en poids, au moins de 15 % en poids ou au moins de 20 % en poids chacun étant basé sur le poids total du produit.

12. Produit de soin pour la peau selon la revendication 10 ou 11 **caractérisé en ce que** le produit est une crème, une lotion pour le corps ou les mains, un maquillage, une lotion tonifiante, un astringent, une composition de nettoyage de la peau, un shampoing, un shampoing antipelliculaire, un après-shampoing capillaire, ou un produit pour le traitement des verrues, de l'acné, d'une dermatite séborrhéique ou d'un psoriasis.

13. Produit de traitement de l'acné selon la revendication 12 dans lequel la concentration de l'acide salicylique est de 0,5 % en poids à 2 % en poids sur la base du poids total du produit de traitement de l'acné.

14. Produit de traitement des verrues selon la revendication 11 dans lequel la concentration de l'acide salicylique est de 5 % en poids à 40 % en poids ou de 17 % en poids à 25 % en poids sur la base du poids total du produit de traitement des verrues.

15. Produit de soin pour la peau selon la revendication 11 sous la forme d'une lotion et dans lequel la concentration de l'acide salicylique est de 0,2 % en poids à 5 % en poids sur la base du poids total du produit.

16. Produit de soin pour la peau selon la revendication 11 sous la forme d'un shampoing antipelliculaire et dans lequel la concentration de l'acide salicylique est de 0,18 % en poids à 3 % en poids sur la base du poids total du produit.

17. Procédé de préparation d'une solution d'acide salicylique comprenant la combinaison du gel d'acide salicylique selon l'une quelconque des revendications 1 à 6 avec un solvant et un mélange.

18. Procédé de préparation d'un produit de soin pour la peau qui comprend une pluralité d'ingrédients comprenant la combinaison des ingrédients avec la solution d'acide salicylique selon la revendication 17 et un mélange.

19. Procédé qui est efficace pour préparer un produit de soin pour la peau qui contient une pluralité d'ingrédients et qui inclut la combinaison des ingrédients avec la solution d'acide salicylique selon la revendication 17 ou avec le gel d'acide salicylique selon la revendication 6 qui ne contient pas de solvant et dans lequel la quantité d'acide salicylique et de stabilisateur comprend pour chacun au moins 30 % en poids du poids total de la solution ou du gel.

20. Procédé selon la revendication 19 dans lequel la quantité d'acide salicylique est de 30 % en poids à 60 % en poids ou de 40 % en poids à 55 % en poids sur la base du poids total de la solution ou du gel.

21. Procédé selon la revendication 19 ou 20 dans lequel le stabilisateur est la cocamine.

22. Procédé selon la revendication 21, dans lequel la quantité de cocamine est de 40 % en poids 70 % en poids ou de 45 % en poids à 60 % en poids et sur la base du poids total de la solution ou du gel.

23. Produit de soin pour la peau préparé selon le procédé de la revendication 19.

24. Produit de soin pour la peau selon la revendication 23 sélectionné dans le groupe constitué par une crème, une lotion pour le corps, une lotion pour les mains, un shampoing antipelliculaire, un médicament contre les verrues, un médicament anti-acné, un maquillage, une lotion tonifiante, un astringent, une composition de nettoyage de la peau, un shampoing, un après-shampoing, une pommade, un produit pour traiter une dermatite séborrhéique et un produit de traitement d'un psoriasis et sur la base du poids du produit :
a) une crème contenant 0,1 % en poids à 5 % en poids d'acide salicylique ;
b) une lotion contenant 0,2 % en poids à 5 % en poids d'acide salicylique ;
c) un maquillage contenant 0,1 % en poids à 3,0 % en poids d'acide salicylique ;
d) un shampoing antipelliculaire contenant 0,1 % en poids à 4 % en poids d'acide salicylique ;
e) un après-shampoing capillaire contenant 0,1 % en poids à 4 % en poids d'acide salicylique
f) un médicament de traitement de l'acné contenant 0,5 % en poids à 2 % en poids d'acide salicylique ;
g) un médicament contre les verrues contenant 5 % en poids à 40 % en poids d'acide salicylique ou 17 % en poids à 25 % en poids d'acide salicylique.
